# FASCICULE DE BREVET EUROPEEN

(11) **EP 1 679 463 B1**
(45) Date de publication et mention de la délivrance du brevet: **14.11.2007**
(21) Numéro de dépôt: 05300936.1
(22) Date de dépôt: 17.11.2005
(51) Int. Cl.: F16L 37/08

(54) **Dispositif régulateur de vide à bocal de sécurité encliquetable utilisable en milieu hospitalier**
Sicherheitsbehälter zur Unterdruckregulierung mit Rastverbindung für den Krankenhausbereich
Safety container for controlling vacuum pressure comprising a snap fit connection for use in a hospital

(30) Priorité: 06.01.2005 FR 0550048
(43) Date de publication de la demande: 12.07.2006
(73) Titulaire: TAEMA, F-92182 Antony Cédex (FR)
(72) Inventeur: Collado, Pedro, 77330, OZOIR LA FERRIERE (FR); Rudnyanin, Philippe, 91310, LONGPONT SUR ORGE (FR); Regnier, Guillaume, 91620, NOZAY (FR)
(74) Mandataire: De Cuenca, Emmanuel Jaime

(56) Documents cités:
- DE-U1- 20 014 909
- DE-U1- 20 115 990
- US-A- 4 123 091
- US-A- 4 747 843

## Description

L'invention concerne un dispositif de régulation de dépression ou de vide, en particulier utilisable dans le domaine médical, comprenant un corps principal et un bocal de sécurité comportant chacun des moyens de couplage/découplage complémentaires permettant leur solidarisation ou désolidarisation aisée et rapide.

Habituellement, les dispositifs de régulation du niveau de vide, encore appelés régulateurs de dépression, venant se connecter sur les prises murales des réseaux de vide au sein des bâtiments hospitaliers ou similaires, sont équipés de moyens permettant l'ouverture ou la fermeture directe de l'alimentation ou de l'arrivée de vide ; la lecture de la dépression (vide), via un vacuomètre indiquant les dépressions de 0 à 1000 mbar ; et la régulation de la pression de vide, typiquement entre 0 et 250 mbar pour certaines applications et entre 0 et 600 mbar pour d'autres applications.

Ces dispositifs, lorsqu'ils sont utilisés dans le milieu médical, sont généralement reliés au réseau de vide de l'hôpital et servent à l'aspiration des déchets et liquides organiques issus des patients, notamment lors des interventions chirurgicales ou analogues.

Les déchets et liquides ainsi aspirés sont récupérés dans un bocal de sécurité fixé à la partie inférieure du dispositif.

Le document DE-U-20115880 est considéré comme l'art de la technique le plus proche.

Or, les systèmes de récupération des déchets organiques mettant en oeuvre de tels dispositifs régulateurs de pression ne sont pas faciles à manipuler, en particulier lors du changement de bocal de sécurité avec ou sans filtre associé, lorsqu'il s'agit d'un ensemble à usage unique, et/ou lors du nettoyage, par voie chimique ou par auto-clavage, de ce bocal lorsqu'il est réutilisable.

En effet, sur les systèmes existants, le bocal est fixé au corps du dispositif généralement par le biais d'un simple pas de vis, c'est-à-dire d'un filetage ou taraudage.

Or, le raccordement par vissage du bocal sur le corps du dispositif de régulation pose souvent problème aux utilisateurs du fait d'une mauvaise prise des filets du filetage et du taraudage complémentaire.

De plus, l'étanchéité au niveau de la connexion n'est efficacement réalisée que si l'utilisateur a correctement connecté et serré l'écrou de serrage du bocal de sécurité, sachant par ailleurs que ceux-ci peuvent, au fil du temps et de leurs manipulations, se desserrer et ne plus réaliser l'étanchéité voulue.

Le problème qui se pose est alors d'améliorer les dispositifs existants et de proposer un dispositif de régulation de vide avec bocal de sécurité comprenant un système de connexion rapide qui réalise l'étanchéité et la connexion en une seule opération, lors de l'accostage entre le régulateur de vide et le bocal, et qui ne puisse pas se desserrer au fil du temps ou de son utilisation de manière à conserver une bonne étanchéité de l'ensemble.

La solution de l'invention est un dispositif régulateur de vide comprenant un corps principal et un bocal de sécurité, ledit bocal et ledit corps comportant chacun des moyens de couplage/découplage complémentaires permettant la solidarisation ou la désolidarisation du bocal sur le corps, caractérisé en ce que les moyens de couplage/découplage comprennent :
- une partie mâle comportant une portion en saillie,
- une partie femelle 8 ayant des dimensions complémentaire de la partie mâle et étant apte à recevoir la partie mâle,
- un logement et
- une pièce mobile,
et en ce que, lors du couplage du bocal au corps principal, la partie mâle vient s'insérer dans la partie femelle, et la pièce mobile vient coopérer avec le logement de manière à obtenir un maintien solidaire du bocal sur le corps principal.

Selon le cas, le dispositif de l'invention peut comprendre l'une ou plusieurs des caractéristiques suivantes :
- la partie mâle est portée par le bocal et la partie femelle est aménagée dans le corps principal.
- la partie mâle est portée par le corps principal et la partie femelle est portée par le bocal.
- le logement est aménagé sur la portion en saillie de la partie mâle et la pièce mobile est portée par la partie femelle.
- la pièce mobile est portée par la portion en saillie de la partie mâle et le logement est aménagé dans la partie femelle.
- le logement est une gorge périphérique aménagée sur au moins une partie de la paroi extérieure de la portion en saillie de la partie mâle.
- la pièce mobile est mobile en translation ou en rotation.
- le corps ou le bocal comprend des moyens de libération coopérant avec la pièce mobile de manière à permettre une désolidarisation du bocal du corps, lors de l'actionnement desdits moyens de libération par un utilisateur.
- les moyens de libération comprennent un bouton-poussoir.
- il comporte un filtre, de préférence le bocal et le filtre forme une structure unique.

L'invention porte aussi sur le bocal de sécurité comportant un réceptacle en forme de coupelle surmonté d'un couvercle muni d'une portion en saillie se projetant vers l'extérieur et formant la partie mâle d'un dispositif selon l'invention, de préférence la portion en saillie comporte un logement aménagé sur sa périphérie externe.

De préférence, la portion en saillie comprend un perçage axial communiquant fluidiquement avec l'intérieur du réceptacle, de préférence le réceptacle comporte un orifice latéral communiquant fluidiquement avec l'intérieur du réceptacle.

L'invention va être mieux comprise grâce à la description détaillée donnée ci-après à titre illustratif, en liaison avec les figures annexées.

Les Figures ci-annexées représentent un dispositif régulateur de vide selon l'invention, lequel est destiné à être relié ou connecté à un réseau de distribution de vide, par exemple dans un bâtiment hospitalier, grâce à un raccord normalisé.

La régulation du niveau de vide se fait par un ensemble interne de régulation de vide classique comprenant un siège, un clapet, une membrane ou un piston, un ressort de détente et une vis de réglage de la pression. De tels moyens de régulation de niveau de vide (ou de dépression) sont identiques à ceux utilisés dans l'art antérieur et ne seront donc pas détaillés davantage ici.

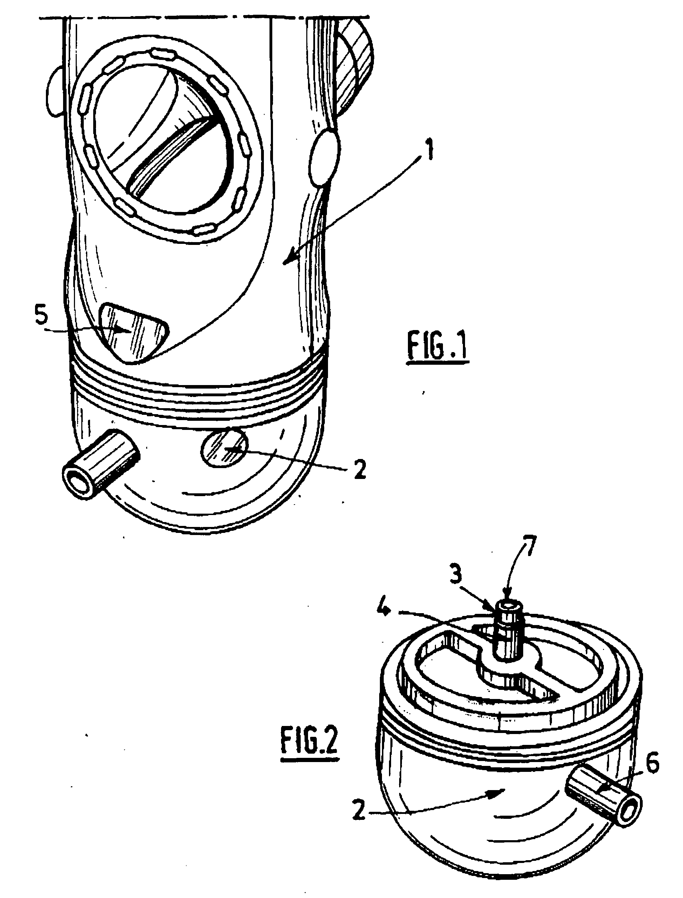

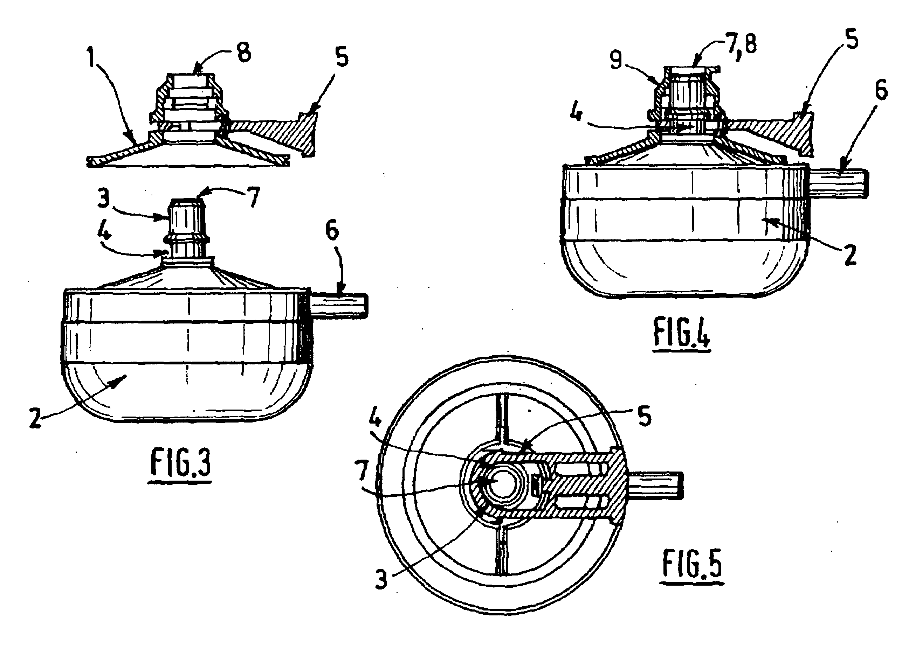

Comme on le voit sur la Figure 1, le dispositif régulateur de vide (vue partielle de la partie inférieure du dispositif) comporte un corps principal 1 sur lequel est fixé un bocal 2 de sécurité situé à sa base formant réceptacle et destiné à recueillir les déchets ou liquides aspirés par le dispositif, lors de son utilisation pendant une opération de soins médicaux.

En outre, un filtre anti-retour peut être incorporé ou non dans le bocal si cela s'avérait nécessaire.

Les Figures 2 à 5 représentent un bocal de sécurité 2 selon l'invention, en vue dissociée du corps 1, dont la partie supérieure se termine par une partie mâle 3, 7 formant une portion en saillie en forme de doigt 3 rainuré 4 permettant un encliquetage du bocal de sécurité 2 dans une partie femelle complémentaire 8 ménagée dans le corps principal 1 du régulateur de la Figure 1.

La portion en saillie 3 comprend un perçage 7 axial faisant communiquer fluidiquement le circuit gaz de la partie femelle 8 du corps 1 avec l'intérieur du réceptacle du bocal 2, lorsqu'ils sont connectés l'un à l'autre. En outre, le réceptacle 2 comporte un orifice latéral 6 communiquant fluidiquement aussi avec l'intérieur du réceptacle et auquel peut être relié un dispositif d'aspiration de fluide, telle une canule ou analogue.

Selon l'invention, une fixation rapide et aisée du bocal de sécurité 2 sur la partie inférieure du corps 1 du dispositif de régulation de vide est obtenue par encliquetage instantané du bocal de sécurité 2 sur le corps 1 du régulateur en exerçant, sur le bocal de sécurité 2, une simple poussée axiale de bas en haut sur la Figure 1, les parties de raccordement mâle 7 et femelle 8 venant alors coopérer l'une avec l'autre pour obtenir la fixation du bocal de sécurité 2 sur le corps du dispositif 1.

Plus précisément, le corps 1 principal du régulateur comporte, dans ce cas, une pièce mobile, tel un cran mobile 5, qui vient coopérer avec le logement 4, par exemple une rainure ou une gorge périphérique, de manière à obtenir un maintien solidaire du bocal de sécurité 2 sur le corps 1 principal.

L'étanchéité peut être réalisée par un joint torique 9 autour du doigt 3 en saillie de la partie mâle.

Pour déconnecter le bocal de sécurité 2, lorsqu'il est nécessaire de le remplacer ou de le nettoyer, il suffit à l'opérateur d'actionner un bouton 5 poussoir de libération aménagé par exemple sur le corps 1, lequel bouton 5 libère le cran mobile par exemple à glissement latéral, ou toute autre pièce mobile en translation ou en rotation, situé ici dans le corps 1, lequel cran mobile vient se loger dans une gorge 4 du doigt 3 du bocal de sécurité 2.

Le bocal de sécurité 2 est alors libéré du fait du désengagement du cran de la gorge 3 et peut être désaccouplé pour son nettoyage et/ou son remplacement.

En outre, le bocal de sécurité se connecte sur la partie inférieure du corps 1 de régulateur grâce à un connecteur rapide disposé sur celui-ci.

Ce montage peut toutefois être inversé, c'est-à-dire que la partie femelle peut se trouver sur le bocal de sécurité 2 et la partie mâle sur le régulateur de vide 1. De même, la pièce mobile et la gorge 4 peuvent se trouver indifféremment sur le bocal de sécurité 2 ou le corps 1.

## Revendications

1. Dispositif régulateur de vide comprenant un corps (1) principal et un bocal (2) de sécurité, ledit bocal (2) et ledit corps (1) comportant chacun des moyens de couplage/découplage complémentaires permettant la solidarisation ou la désolidarisation du bocal (2) sur le corps (1), **caractérisé en ce que** les moyens de couplage/découplage comprennent :
- une partie mâle (3, 4, 7) comportant une portion en saillie (3),
- une partie femelle (8) ayant des dimensions complémentaire de la partie mâle (3, 4, 7) et étant apte à recevoir la partie mâle (3, 4, 7),
- un logement (4) et
- une pièce mobile,
et **en ce que**, lors du couplage du bocal (2) au corps (1) principal, la partie mâle (3, 4, 7) vient s'insérer dans la partie femelle (8), et la pièce mobile (5) vient coopérer avec le logement (4) de manière à obtenir un maintien solidaire du bocal (2) sur le corps (1) principal.

2. Dispositif selon la revendication 1, **caractérisé en ce que** la partie mâle (3, 4, 7) est portée par le bocal (2) et la partie femelle (8) est aménagée dans le corps (1) principal.

3. Dispositif selon la revendication 1, **caractérisé en ce que** la partie mâle (3, 4, 7) est portée par le corps (1) principal et la partie femelle (8) est portée par le bocal (2).

4. Dispositif selon l'une des revendications 1 à 3, **caractérisé en ce que** le logement (4) est aménagé sur la portion en saillie (3) de la partie mâle (3, 4, 7) et la pièce mobile (5) est portée par la partie femelle (8).

5. Dispositif selon l'une des revendications 1 à 3, **caractérisé en ce que** la pièce mobile est portée par la portion en saillie (3) de la partie mâle (3, 4, 7) et le logement (4) est aménagé dans la partie femelle (8).

6. Dispositif selon l'une des revendications 1 à 4, **caractérisé en ce que** le logement (4) est une gorge périphérique aménagée sur au moins une partie de la paroi extérieure de la portion en saillie (3) de la partie mâle (3, 4, 7).

7. Dispositif selon l'une des revendications 1 à 6, **caractérisé en ce que** la pièce mobile (5) est mobile en translation ou en rotation.

8. Dispositif selon l'une des revendications 1 à 7, **caractérisé en ce que** le corps (1) ou le bocal (2) comprend des moyens de libération (5) coopérant avec la pièce mobile de manière à permettre une désolidarisation du bocal (2) du corps (1), lors de l'actionnement desdits moyens de libération par un utilisateur.

9. Dispositif selon la revendication 8, **caractérisé en ce que** les moyens de libération (5) comprennent un bouton-poussoir (5).

10. Dispositif selon l'une des revendications 1 à 7, **caractérisé en ce qu**'il comporte un filtre, de préférence le bocal (2) et le filtre forme une structure unique.

11. Bocal (2) de sécurité comportant un réceptacle en forme de coupelle surmonté d'un couvercle muni d'une portion en saillie (3) se projetant vers l'extérieur et formant la partie mâle (3, 4, 7) d'un dispositif selon l'une des revendications 1 à 10, de préférence la portion en saillie (3) comporte un logement (4) aménagé sur sa périphérie externe.

12. Bocal selon la revendication 11, **caractérisé en ce que** la portion en saillie (3) comprend un perçage (7) axial communiquant fluidiquement avec l'intérieur du réceptacle, de préférence le réceptacle comporte un orifice latéral (6) communiquant fluidiquement avec l'intérieur du réceptacle.

## Claims

1. Vacuum regulator device comprising a main body (1) and a safety container (2), the said container (2) and the said body (1) each comprising complementary coupling/uncoupling means allowing the container (2) to be secured to or detached from the body (1), **characterized in that** the coupling/uncoupling means comprise:
- a male part (3, 4, 7) comprising a projecting portion (3),
- a female part (8) having dimensions that complement those of the male part (3, 4, 7) and which is able to accept the male part (3, 4, 7) ,
- a housing (4), and
- a moving part,
and **in that**, when the container (2) is being coupled to the main body (1), the male part (3, 4, 7) is inserted in the female part (8), and the moving part (5) collaborates with the housing (4) in such a way as to hold the container (2) securely on the main body (1).

2. Device according to Claim 1, **characterized in that** the male part (3, 4, 7) is borne by the container (2) and the female part (8) is formed in the main body (1).

3. Device according to Claim 1, **characterized in that** the male part (3, 4, 7) is borne by the main body (1) and the female part (8) is borne by the container (2).

4. Device according to one of Claims 1 to 3, **characterized in that** the housing (4) is formed on the projecting portion (3) of the male part (3, 4, 7) and the moving part (5) is borne by the female part (8).

5. Device according to one of Claims 1 to 3, **characterized in that** the moving part is borne by the projecting portion (3) of the male part (3, 4, 7) and the housing (4) is formed in the female part (8).

6. Device according to one of Claims 1 to 4, **characterized in that** the housing (4) is a peripheral groove formed on at least part of the exterior wall of the projecting portion (3) of the male part (3, 4, 7).

7. Device according to one of Claims 1 to 6, **characterized in that** the moving part (5) is able to move in terms of translation or in terms of rotation.

8. Device according to one of Claims 1 to 7, **characterized in that** the body (1) or the container (2) comprises release means (5) collaborating with the moving part in such a way as to allow the container (2) to be detached from the body (1) when the said release means are actuated by a user.

9. Device according to Claim 8, **characterized in that** the release means (5) comprise a push-button (5).

10. Device according to one of Claims 1 to 7, **characterized in that** it comprises a filter, and the container (2) and filter preferably form a single structure.

11. Safety container (2) comprising a cup-shaped receptacle surmounted by a lid equipped with a projecting portion (3) protruding outwards and forming the male part (3, 4, 7) of a device according to one of Claims 1 to 10, the projecting portion (3) preferably comprising a housing (4) formed on its external periphery.

12. Container according to Claim 11, **characterized in that** the projecting portion (3) comprises an axial drilling (7) in fluidic communication with the inside of the receptacle, the receptacle preferably comprising a lateral orifice (6) in fluidic communication with the inside of the receptacle.

## Patentansprüche

1. Vakuumregelvorrichtung, die einen Hauptkörper (1) und einen Sicherheitsbehälter (2) aufweist, wobei der Behälter (2) und der Körper (1) jeder ergänzende Mittel zum Koppeln/Abkoppeln aufweisen, die das Befestigen oder das Trennen des Behälters (2) auf/von dem Körper (1) erlauben, **dadurch gekennzeichnet, dass** die Koppel-/Abkoppelmittel Folgendes aufweisen:
- einen Steckteil (3, 4, 7), der einen vorspringenden Abschnitt (3) aufweist,
- einen Buchsenteil (8), der zu dem Steckteil (3, 4, 7) ergänzende Maße hat und den Steckteil (3, 4, 7) aufnehmen kann,
- eine Aufnahme (4) und
- einen beweglichen Teil,
und **dadurch**, dass sich der Steckteil (3, 4, 7) beim Koppeln des Behälters (2) an den Hauptkörper (1) in den Buchsenteil (8) fügt, und der bewegliche Teil (5) mit der Aufnahme (4) derart zusammenwirkt, dass ein festes Halten des Behälters (2) auf dem Hauptkörper (1) erzielt wird.

2. Vorrichtung nach Anspruch 1, **dadurch gekennzeichnet, dass** der Steckteil (3, 4, 7) von dem Behälter (2) getragen wird, und dass der bewegliche Teil (8) in dem Hauptkörper (1) eingerichtet ist.

3. Vorrichtung nach Anspruch 1, **dadurch gekennzeichnet, dass** der Steckteil (3, 4; 7) von dem Hauptkörper (1) getragen wird, und dass der Buchsenteil (8) von dem Behälter (2) getragen wird.

4. Vorrichtung nach einem der Ansprüche 1 bis 3, **dadurch gekennzeichnet, dass** die Aufnahme (4) auf dem vorspringenden Abschnitt (3) des Steckteils (3, 4, 7) eingerichtet ist, und dass der bewegliche Teil (5) von dem Buchsenteil (8) getragen wird.

5. Vorrichtung nach einem der Ansprüche 1 bis 3, **dadurch gekennzeichnet, dass** der bewegliche Teil von dem vorspringenden Abschnitt (3) des Steckteils (3, 4, 7) getragen wird, und dass die Aufnahme (4) in dem Buchsenteil (8) eingerichtet ist.

6. Vorrichtung nach einem der Ansprüche 1 bis 4, **dadurch gekennzeichnet, dass** die Aufnahme (4) eine umfängliche Hohlkehle ist, die auf mindestens einem Teil der Außenwand des vorspringenden Abschnitts (3) des Steckteils (3, 4, 7) eingerichtet ist.

7. Vorrichtung nach einem der Ansprüche 1 bis 6, **dadurch gekennzeichnet, dass** der bewegliche Teil (5) in Verschiebung oder in Drehung beweglich ist.

8. Vorrichtung nach einem der Ansprüche 1 bis 7, **dadurch gekennzeichnet, dass** der Körper (1) oder der Behälter (2) Freigabemittel (5) aufweist, die mit dem beweglichen Teil derart zusammenarbeiten, dass ein Trennen des Behälters (2) von dem Körper (1) beim Betätigen der Freigabemittel durch einen Benutzer gestattet wird.

9. Vorrichtung nach Anspruch 8, **dadurch gekennzeichnet, dass** die Freigabemittel (5) einen Druckknopf (5) aufweisen.

10. Vorrichtung nach einem der Ansprüche 1 bis 7, **dadurch gekennzeichnet, dass** sie einen Filter aufweist, und dass der Behälter (2) und der Filter vorzugsweise eine einteilige Struktur bilden.

11. Sicherheitsbehälter (2), der eine Aufnahme in Schalenform aufweist, über der ein Deckel liegt, der mit einem vorspringenden Abschnitt (3) versehen ist, der nach außen vorsteht und den Steckteil (3, 4, 7) einer Vorrichtung nach einem der Ansprüche 1 bis 10 bildet, wobei der vorspringende Abschnitt (3) vorzugsweise eine Aufnahme (4) auf seinem äußeren Umfang eingerichtet aufweist.

12. Behälter nach Anspruch 11, **dadurch gekennzeichnet, dass** der vorspringende Abschnitt (3) eine axiale Bohrung (7) aufweist, die fluidisch mit dem Inneren der Aufnahme kommuniziert, wobei die Aufnahme vorzugsweise eine seitliche Öffnung (6) aufweist, die fluidisch mit dem Inneren der Aufnahme kommuniziert.
